(19) Europäisches Patentamt
European Patent Office
Office européen des brevets

(11) Veröffentlichungsnummer: **0 013 749**
**B1**

(12) # EUROPÄISCHE PATENTSCHRIFT

(45) Veröffentlichungstag der Patentschrift: **12.05.82**

(51) Int. Cl.³: **C 07 D 267/10,** A 61 K 31/55 // C07C93/14, C07C97/10, C07C103/38

(21) Anmeldenummer: **79105324.2**

(22) Anmeldetag: **21.12.79**

(54) **Hexahydro-1,4-oxazepine, Verfahren zu deren Herstellung und diese enthaltende Arzneimittel.**

(30) Priorität: **13.01.79 DE 2901180**

(43) Veröffentlichungstag der Anmeldung: **06.08.80 Patentblatt 80/16**

(45) Bekanntmachung des Hinweises auf die Patenterteilung: **12.05.82 Patentblatt 82/19**

(84) Benannte Vertragsstaaten: **AT BE CH DE FR GB IT LU NL SE**

(56) Entgegenhaltungen:
**DE-A-1 941 534**
**US-A-3 391 149**
**»Liebigs Annalen der Chemie«, Band 10, Oktober 1976, Weinheim, DE, H. GRIENGL et al.: »7-Alkoxy-hexahydro-1,4-oxazepine«, Seiten 1792—98**

(73) Patentinhaber: **BASF Aktiengesellschaft, Carl-Bosch-Strasse 38, D-6700 Ludwigshafen (DE)**

(72) Erfinder: **Treiber, Hans Joerg, Dr., Sperberweg 1, 6831 Bruehl (DE)**
Erfinder: **Lenke, Dieter, Dr., Kekuleplatz 1, D-6700 Ludwigshafen (DE)**
Erfinder: **Worstmann, Wolfgang, Dr., Am Bildstock 11, D-6718 Gruenstadt 1 (DE)**

EP 0 013 749 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Hexahydro-1,4-oxazepine, Verfahren zu deren Herstellung und diese enthaltende Arzneimittel

Es ist bekannt, daß bestimmte stickstoffhaltige 7- und 8-Ring-Heterocyclen analgetische Wirkung aufweisen. So sind aus der Gruppe der Azepine Meptazinol (3-(3-Ethylhexahydro-1-methyl-1H-azepin-3-yl)-phenol, DE-AS 19 41 534) und aus der Gruppe der Benzoxazocine Nefopam (5-Methyl-1-phenyl-3,4,5,6-tetrahydro-1H-benz[f]-2,5-oxazocin, DE-C-16 20 198) als Analgetica bekannt.

Es wurden nun neue Oxazepin-Derivate gefunden, die eine stärkere analgetische Wirkung zeigen.

Gegenstand der Erfindung sind Hexahydro-1,4-oxazepine der allgemeinen Formel I

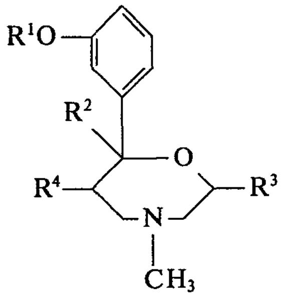

(I)

worin

| | |
|---|---|
| $R^1$ | ein Wasserstoffatom oder eine Alkyl- oder Acylgruppe mit jeweils 1 bis 4 Kohlenstoffatomen, |
| $R^2$ | eine Alkylgruppe mit 1 bis 4 Kohlenstoffatomen und |
| $R^3$ und $R^4$ | gleich oder verschieden sind und Wasserstoffatome oder Methylgruppen bedeuten, |

sowie deren Salze mit physiologisch verträglichen Säuren.

Gegenstand der Erfindung ist weiter ein Verfahren zur Herstellung der Hexahydro-1,4-oxazepine der allgemeinen Formel I, welches darin besteht, daß man eine Verbindung der allgemeinen Formel II

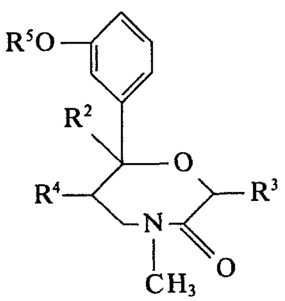

(II)

worin

$R^5$ eine Alkylgruppe mit 1 bis 4 Kohlenstoffatomen bedeutet und $R^2$, $R^3$ und $R^4$ die vorstehende Bedeutung haben,

reduziert und anschließend gegebenenfalls die Alkylgruppe $R^5$ gegen ein Wasserstoffatom oder eine Acylgruppe austauscht und die so erhaltenen Verbindungen, falls gewünscht, in ihre Salze mit physiologisch verträglichen Säuren überführt.

Schließlich betrifft die Erfindung auch Arzneimittel, welche Hexahydro-1,4-oxazepine der allgemeinen Formel I enthalten.

Die Verbindungen I enthalten 1 bis 3 asymmetrische Kohlenstoffatome, so daß die Verbindungen in Form von Racematen oder Stereoisomeren dargestellt werden können. Die Stereoisomeren können sowohl durch asymmetrische Synthese als auch durch Trennung der Racemate rein erhalten werden.

Zur Reduktion der Verbindungen II benötigt man starke Reduktionsmittel wie Diboran oder vorzugsweise Lithiumaluminiumhydrid, wobei als Lösungsmittel besonders Tetrahydrofuran, Dioxan oder Ether geeignet sind. Die Reduktion wird bei erhöhter Temperatur vorzugsweise bei Siedetemperatur des Lösungsmittels durchgeführt.

Der Austausch einer Alkoxygruppe am Phenylring gegen eine Hydroxygruppe kann z. B. mit basischen, etherspaltenden Verbindungen wie Natriummethylmercaptid in einem dipolar aprotischen Lösungsmittel wie Hexamethylphosphorsäuretriamid, Dimethylsulfoxid oder Dimethylformamid bei 50 bis 200° C, vorzugsweise bei 100 bis 150° C erfolgen.

Zur Acylierung der freien Hydroxygruppen können praktisch alle bekannten Verfahren

herangezogen werden. Am einfachsten ist die Umsetzung mit den Säureanhydriden oder -halogeniden bei erhöhter Temperatur.

Die zur Herstellung der neuen Verbindungen benötigten Ausgangsstoffe der allgemeinen Formel II sind noch nicht beschrieben. Sie lassen sich wie folgt darstellen:

Durch Umsetzen der Ketone III mit N-Benzyl-N-methyl-methyllenimoniumchlorid IV erhält man sogenannte Mannich-Verbindungen V (vgl. Angew. Chem. 88, 261 (1976)):

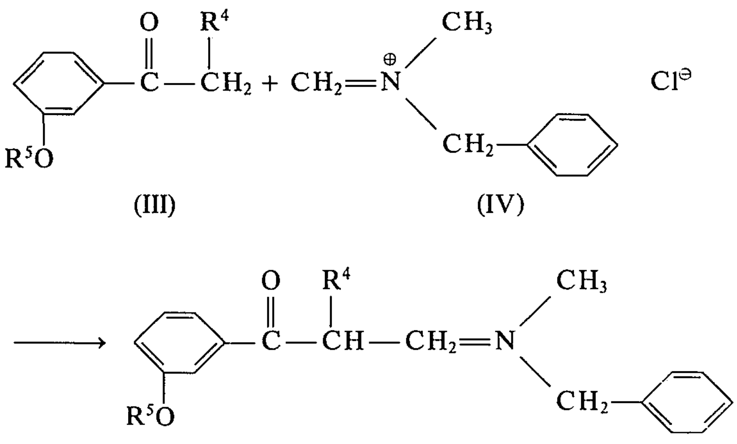

(III) (IV)

(V)

Die Verbindungen V reagieren mit Alkylgridnard-Verbindungen zu den Verbindungen VI (vgl. J. Amer. Chem. Soc., 71, 2050 (1948))

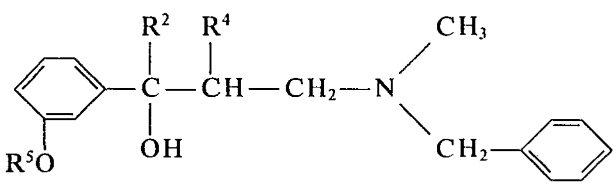

(VI)

aus denen der Benzylrest abhydriert wird.

Aus den debenzylierten Verbindungen erhält man durch Umsetzen mit Chloracetylchlorid oder $\alpha$-Chlorpropionylchlorid in Gegenwart von verdünnter Natronlauge oder Triethylamin die Verbindungen

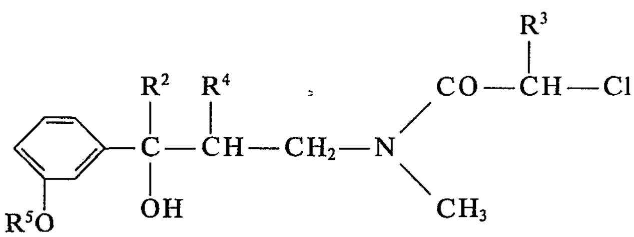

(VII)

aus denen sich durch Erwärmen mit einer Base wie Kalium-tert-butanolat in Dimethylsulfoxid die Verbindungen II herstellen lassen (vgl. DE-A-1 944 468).

Die erfindungsgemäßen Substanzen zeichnen sich durch eine ausgeprägte analgetische Wirkung aus. Sie eignen sich daher zur Pharmakotherapie von Schmerzzuständen verschiedener Ursache.

Als Modell zur Prüfung der analgetischen Wirkung diente der sogenannte Brennstrahl-Test nach D'AMOUR und SMITH (J. Pharmacol. 72, 74—79, 1941). In diesem Experiment werden die zu prüfenden Substanzen (wäßrige Lösungen; Injektionsvolumen 10 ml/kg) Gruppen von 10 weiblichen Mäusen (Stamm: NMRI) im Gewicht von 19 bis 23 g intraperitoneal oder oral appliziert.

Schmerzreaktionen werden durch thermische Reizung (Fokussierte Wärmebestrahlung des Schwanzes mit einer Halogenlampe für maximal 30 sec.) vor und 30 min nach der Substanz-Applikation ausgelöst.

Die Zeit bis zum reflektorischen Wegziehen des Schwanzes aus der Bestrahlungszone wird als Reaktionszeit gemessen. Sie beträgt bei 670 unbehandelten Tieren $6{,}5 \pm 0{,}29$ sec. Analgetisch wirkende Substanzen verlängern die Reaktionszeit dosisabhängig. Zwischen den Logarithmen der Dosen (mg/kg) und der relativen Verlängerung der Reaktionszeit ($\Delta$ %) besteht eine lineare Beziehung, aus der mit Hilfe der Regressionsanalyse als ED 100% die Dosis berechnet wird, welche die

Reaktionszeit verdoppelt. Bei einer Bestrahlungsdauer von maximal 30 sec beträgt die größtmögliche Verlängerung der Reaktionszeit rund 360%.

Die schmerzliche Wirkung der erfindungsgemäßen Substanzen im Brennstrahl-Test tritt besonders bei der klinisch bedeutsamen oralen Applikation hervor (Tab. 1). Die Wirksamkeit übertrifft hier die von Nefopam um das 2- bis 28fache. Der als Maß für die enterale Wirksamkeit zu verwendende Quotient aus der wirksamen Dosis (ED 100%) bei intraperitonealer und oraler Applikation (0,41 bis 0,83) ist sehr hoch. Er zeigt an, daß die oral wirksamen Dosen der erfindungsgemäßen Substanzen nur wenig über den i. p.-wirksamen liegen. Die enterale Wirksamkeit ist 2,4- bis 4,9mal größer als die des Nefopam.

Im Brennstrahl-Test kann mit nichttoxischen Dosen Nefopam unabhängig von der Applikationsart nur eine partielle Analgesie (192% Verlängerung der Reaktionszeit bei i. p. Applikation, 93% bei oraler Gabe) erzielt werden. Bei höherer Dosierung wirkt Nefopam toxisch (hohe Todesrate).

Mit den erfindungsgemäßen Substanzen läßt sich dagegen die Reaktionszeit auf wesentlich höhere Maximalwerte verlängern (um 276 bis 435% bei i. p. Applikation bzw. um 223 bis 419% bei oraler Applikation), ohne daß toxische Wirkungen auftreten.

Tabelle 1: Analgetische Wirkung. Brennstrahl-Test. Maus

| Substanz des Beispiels Nr. | Applikation i.p. | | | | Applikation per os | | | | Q[3]) |
|---|---|---|---|---|---|---|---|---|---|
| | ED 100%[1]) | Relative Wirksamkeit | Max. Wirkung[2]) mg/kg | % | ED 100% | Relative Wirksamkeit | Max. Wirkung mg/kg | % | |
| 2 | 12,7 | 0,64 | 46,4 | 332 | 22,4 | 2,07 | 100 | 333 | 0,57 |
| 1 | 1,71 | 4,73 | 10,0 | 276 | 2,07 | 22,42 | 10,0 | 387 | 0,83 |
| 6 | 6,83 | 1,18 | 46,4 | 326 | 8,71 | 5,33 | 46,4 | 419 | 0,78 |
| 7 | 0,675 | 11,97 | 4,64 | 382 | 1,66 | 27,95 | 4,64 | 300 | 0,41 |
| 3 | 6,58 | 1,23 | 21,5 | 351 | 15,3 | 3,03 | 46,4 | 223 | 0,43 |
| 4 | 4,43 | 1,82 | 21,5 | 319 | 6,27 | 7,40 | 100 | 397 | 0,71 |
| 5 | 5,66 | 1,43 | 46,4 | 435 | 7,01 | 6,62 | 21,5 | 318 | 0,81 |
| 9 (+) | 2,08 | 3,89 | 10,0 | 339 | 2,76 | 16,81 | 10,0 | 310 | 0,75 |
| Nefopam | 8,08 | = 1,00 | 21,5[4]) | 192 | ca 46,4 | = 1,00 | 46,4[5]) | 93 | 0,17 |

[1]) Dosis (mg/kg), welche die Reaktionszeit um 100% verlängert.

[2]) Größtmögliche Verlängerung der Reaktionszeit bei einem Dosenabstand von $\sqrt[3]{10}$.

[3]) Q = Enterale Wirksamkeit = ED 100% i.p./ED 100% per os.

[4]) Bei 46,4 mg/kg toxisch (6 von 10 Tieren gestorben).

[5]) Bei 100 mg/kg toxisch (6 von 10 Tieren gestorben).

Die erfindungsgemäßen Verbindungen können in üblicher Weise oral oder parenteral (subkutan, intravenös, intramuskular, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 0,01 bis 1,0 mg/kg pro Patient sowohl bei intravenöser, subkutaner oder intramuskulärer als auch bei oraler Anwendung. Die Applikation dieser Dosis erfolgt 1 bis 3× täglich. In schweren Fällen kann auch noch öfter appliziert werden.

Die neuen Verbindungen können in den gebräuchlichen galenischen Applikationsformen fest oder flüssig angewendet werden, z. B. als Tabletten, Kapseln, Pulver, Granulate, Dragees, Lösungen oder Suppositorien. Diese werden in üblicher Weise hergestellt. Die Wirkstoffe können dabei mit den üblichen galenischen Hilfsmitteln wie Tablettenbindern, Füllstoffen, Konservierungsmitteln,

Tablettensprengmitteln, Fließreguliermitteln, Weichmachern, Netzmitteln, Dispergiermitteln, Emulgatoren, Lösungsmitteln, Retardierungsmitteln und/oder Antioxodantien verarbeitet werden (vgl. L. G. Goodman, A. Gilman: The Pharmacological Basis of therapeutics).

Die neuen Substanzen können auch in Form ihrer Salze mit physiologisch verträglichen Säuren verabfolgt werden. Solche Säuren sind beispielsweise Salzsäure, Schwefelsäure, Phosphorsäure, Weinsäure, Zitronensäure, Fumarsäure, Essigsäure, Ameisensäure, Bernsteinsäure, Maleinsäure, Milchsäure, Amidosulfonsäure.

Herstellung der Ausgangsstoffe

a) β-(N-Benzyl-N-methyl)-amino-3-methoxypropiophenon-hydrochlorid

60 g 3-Methoxyacetophenon (0,4 Mol) werden mit 70 g N-Benzyl-N-methyl-methylenimoniumchlorid (0,4 Mol) (hergestellt aus Bis-(benzylmethylamino)methan und Acetylchlorid) in 500 ml trockenem Acetonitril unter Rühren 60 Minuten auf 75° C und 15 Minuten auf 80° C erwärmt. Man kühlt ab, gibt die Lösung in 2 l Ether, saugt ab und erhält 88 g (69% der Theorie) Produkt vom Schmelzpunkt 134 bis 138° C, das aus Isopronanol umkristallisiert oder für die nachfolgende Umsetzung roh verwendet werden kann.

Fp. aus Isopropanol 140 bis 142° C.

Ebenso wird erhalten:

β-(N-Benzyl-N-methyl)-amino-α-methyl-3-methoxy-propiophenonhydrochlorid aus 3-Methoxypropiophenon.
Fp. 125° C.

b) 1-(N-Benzyl-N-methyl)amino-3-hydroxy-3-(3-methoxyphenyl)-pentan

In eine Grignardlösung, die man aus 218 g (2,0 Mol) Ethylbromid, 48 g (2,0 Mol) Magnesium und 1,5 l trockenem Ether bereitet hat, trägt man unter Rühren und Eisbadkühlung 170 g (0,53 Mol) β-(N-Benzyl-N-methyl)-amino-3-methoxypropiophenon-hydrochlorid (erhalten gemäß a)) ein, erwärmt 2 bis 3 Stunden zum Sieden unter Rückfluß, läßt über Nacht bei Raumtemperatur nachrühren und zersetzt den Ansatz mit Ammoniumchloridlösung. Die Etherphase wird abgetrennt, mit Natriumsulfat getrocknet, der Ether abdestilliert und der Rückstand im Vakuum fraktioniert.

Ausbeute 134 g (81% der Theorie).
$Kp_{0,01\ mbar}$ 180—190° C.

Ebenso werden erhalten:

1-(N-Benzyl-N-methyl)-amino-3-hydroxy-3-(3-methoxyphenyl)butan $Kp_{0,01\ mbar}$ 175 bis 180° C,
1-(N-Benzyl-N-methyl)amino-3-hydroxy-3-(3-methoxyphenyl)hexan $Kp_{0,01\ mbar}$ 185 bis 195° C,
1-(N-Benzyl-N-methyl)amino-2-methyl-3-hydroxy-3-(3-methoxyphenyl)pentan $Kp_{0,01\ mbar}$ 180 bis 185° C.

c) 1-Methylamino-3-hydroxy-3-(3-methoxyphenyl)-pentan

78,6 g (0,25 Mol) 1-(N-Benzyl-N-methyl)amino-3-hydroxy-3-(3-methoxyphenyl)-pentan (hergestellt gemäß b)) werden in 400 ml Methanol gelöst und mit 8 g 10%igem Palladiumkohle-Katalysator bei Normaldruck und Raumtemperatur hydriert. Nach Beendigung der Wasserstoffaufnahme saugt man vom Katalysator ab, dampft die Lösung ein und erhält das Produkt als kristallisierendes Öl.

Ausbeute: 53 g (94% der Theorie).
Fp. 52 bis 54° C aus Hexan.

Ebenso werden erhalten:

1-Methylamino-3-hydroxy-3-(3-methoxyphenyl)butan
(roh weiterverarbeitet)
1-Methylamino-3-hydroxy-3-(3-methoxyphenyl)hexan
Fp. 70 bis 72° C aus Hexan

1-Methylamino-2-methyl-3-hydroxy-3-(3-methoxyphenyl)-pentan (roh weiterverarbeitet).

d) 1-(N-Chloracetyl)-methylamino-3-hydroxy-3-(3-methoxyphenyl)pentan

Zu einer Lösung von 35 g (0,16 Mol) 1-(Methylamino-3-hydroxy-3-(3-methoxyphenyl)pentan (erhalten gemäß c)) in 250 ml Ether gibt man 100 ml 2-N-Natronlauge und tropft dann innerhalb von 30 min unter Rühren 18 g (0,16 Mol) Chloracetylchlorid zu. Man erwärmt anschließend 90 min, trennt die Etherschicht ab, trocknet mit Natriumsulfat und destilliert das Lösungsmittel ab. Der Rückstand wird roh weiterverarbeitet.

Ebenso werden erhalten:

1-(N-Chloracetyl)methylamino-3-hydroxy-3-(3-methoxyphenyl)-butan,
1-(N-Chloracetyl)methylamino-3-hydroxy-3-(3-methoxyphenyl)-hexan,
1-(N-Chloracetyl)methylamino-2-methyl-3-hydroxy-3-(3-methoxyphenyl)pentan.

Unter Verwendung von α-Chlorpropionylchlorid erhält man
1-(N-α-Chlorpropionyl)-methylamino-3-hydroxy-3-(3-methoxyphenyl)pentan.

e) 7-Ethyl-7-(3-methoxyphenyl)-4-methyl-hexahydro-1,4-oxazepin-3-on

41,4 g (0,14 Mol) 1(N-Chloracetyl)methylamino-3-hydroxy-3-(3-methoxyphenyl)pentan, erhalten gemäß d), werden in 200 ml Dimethylsulfoxid gelöst und unter Rühren und leichter Kühlung bei 20°C 33,6 g Kalium-tert-butanolat portionsweise zugefügt. Man erwärmt anschließend 30 bis 120 min auf 50°C und läßt anschließend bei Raumtemperatur über Nacht weiterrühren. Zur Aufarbeitung wird entweder das Lösungsmittel bei möglichst niederer Temperatur im Vakuum abdestilliert, oder der Ansatz mit 1,5 l Wasser verdünnt und mit 3 × 250 ml Methylenchlorid extrahiert. Durch Trocknen über Natriumsulfat wird das Lösungsmittel entfernt und das Rohprodukt erhalten, das direkt weiterverarbeitet wird.

Ebenso werden erhalten:

4,7-Dimethyl-7-(3-methoxyphenyl)-hexahydro-1,4-oxazepin-3-on; wird kristallin, Fp. 88—94° C,
7-(3-Methoxyphenyl)-4-methyl-7-propyl-hexahydro-1,4-oxazepin-3-on,
4,6-Dimethyl-7-ethyl-7-(3-methoxyphenyl)-hexahydro-1,4-oxazepin-3-on,
2,4-Dimethyl-7-ethyl-7-(3-methoxyphenyl)-hexahydro-1,4-oxazepin-3-on.

## Herstellung der Endprodukte

### Beispiel 1

7-Ethyl-7-(3-methoxyphenyl)-4-methyl-hexahydro-1,4-oxazepin

36 g (0,14 Mol) rohes 7-Ethyl-7-(3-methoxyphenyl)-4-methylhexahydro-1,4-oxazepin (vgl. e) werden in 100 ml absolutem Tetrahydrofuran gelöst und bei Siedetemperatur zu einer Suspension von 15 g Lithiumaluminiumhydrid in 500 ml absolutem Tetrahydrofuran zugetropft. Man erwärmt anschließend 6 Stunden auf Siedetemperatur, kühlt ab, zersetzt in üblicher Weise mit Wasser und erhält nach Absaugen des anorganischen Rückstandes, Trocknen und Abdestillieren des Lösungsmittels die Rohbase, die mit isopropanolischer Salzsäure in ihr Hydrochlorid überführt wird.

Ausbeute an Hydrochlorid 15 g (38% der Theorie),
Fp. 181—183°C.

Ebenso werden die nachstehenden, tabellarisch aufgeführten Verbindungen erhalten:

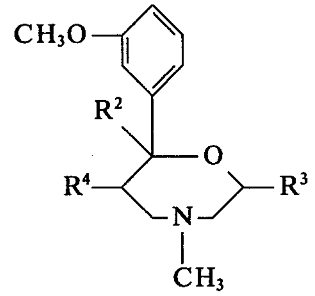

| Beispiel Nr. | $R^2$ | $R^3$ | $R^4$ | Hydrochlorid Fp. °C | Ausbeute % |
|---|---|---|---|---|---|
| 2 | $CH_3$ | H | H | 207–208 | 32 |
| 3 | $C_3H_7$ | H | H | 199–201 | 33 |
| 4 | $C_2H_5$ | $CH_3$ | H | 185–187 | 28 |
| 5 | $C_2H_5$ | H | $CH_3$ | 202–203 | 47 |

Beispiel 6

4,7-Dimethyl-7-(3-hydroxyphenyl)-hexahydro-1,4-oxazepin

Man stellt aus 2,3 g (0,1 Mol) Natrium, 100 ml absolutem Ethanol und 6,2 g (0,1 Mol) Ethylmercaptan eine ethanolische Lösung von Natriummethylmercaptid dar, destilliert im Vakuum den Alkohol ab, fügt 50 ml trockenes Dimethylformamid und 5,1 g (0,02 Mol) 4,7-Dimethyl-7-(3-methoxyphenyl)-hexahydro-1,4-oxazepin (Base) (erhalten nach Beispiel 1) hinzu und erhitzt 3 Stunden auf 140° C.

Anschließend verdünnt man mit 500 ml Wasser, neutralisiert mit Essigsäure und extrahiert die Lösung mehrmals mit Methylenchlorid. Nach Entfernen des Lösungsmittels nimmt man in 100 ml Ether auf und fällt das Produkt als Hydrochlorid durch Einleiten von Chlorwasserstoffgas. Es wird aus Ethanol umkristallisiert.

Ausbeute: 2,7 g (53% der Theorie).
Fp. 248° C

Ebenso werden erhalten:

Beispiel 7

7-Ethyl-7-(3-hydroxyphenyl)-4-methyl-hexahydro-1,4-oxazepin, Fp. 204–206° C.

Beispiel 8

7-(3-Acetoxyphenyl)-7-ethyl-4-methyl-hexahydro-1,4-oxazepin

2,7 g (0,01 Mol) des nach Beispiel 2 erhaltenen 7-Ethyl-7-(3-Hydroxyphenyl)-4-methyl-hexahydro-1,4-oxazepin-hydrochlorids werden mit 25 ml Essigsäureanhydrid 3 Stunden zum Sieden erhitzt. Man destilliert anschließend das überschüssige Essigsäureanhydrid im Vakuum ab und kristallisiert den Rückstand aus Isopropanol-Ether um.

Ausbeute 2,4 g des Hydrochlorids (75% der Theorie).
Fp. 210° C.

Beispiel 9

(+)- bzw. (–)-7-Ethyl-7-(3-methoxyphenyl)-4-methyl-hexahydro-1,4-oxazepin (Racematspaltung)

Aus einer Lösung von 12 g (0,05 Mol) racemischem 7-Ethyl-7-(3-methoxyphenyl)-4-methyl-hexahydro-1,4-oxazepin (vgl. Beispiel 1) und 17 g (0,05 Mol) L(–)-0,0-Dibenzoylweinsäuremonohydrat in 50 ml Isopropanol und 10 ml Diisopropylether kristallisiert nach einiger Zeit das Salz der rechtsdrehenden Base aus. Nach 3- bis 4maligem Umkristallisieren aus der 5fachen Menge Isopropanol erhält man 6 g (ca. 40% der Theorie) drehwertkonstantes Produkt.

Spezifischer Drehwert:
$[\alpha]^{20}_{589\,nm} = -45°$. (c = 10 ml/ml in Ethanol).

Aus dem Salz erhält man in bekannter Weise die Base, die in ihr Hydrochlorid überführt wird.

Verwendet man für die Racemat-Spaltung D-(+)-0,0-Dibenzoylweinsäure, so wird analog das Dibenzoyltartrat der linksdrehenden Base erhalten.

Spezifischer Drehwerte:
Basen: $[\alpha]^{20}_{589} = +/-35°$ —(c=28 mg/ml Ethanol)
Hydrochloride: $[\alpha]^{20}_{589} = +/-44°$ (c=10 mg/ml Ethanol)
Fp. 202—203° C.

Beispiel 10

Auf einer Tablettenpresse werden in üblicher Weise Tabletten folgender Zusammensetzung gepreßt:

10,00 mg 7-Ethyl-7-(3-methoxyphenyl)-4-methyl-hexahydro-1,4-oxazepin-hydrochlorid
50,00 mg Maisstärke
4,50 mg Gelatine
15,00 mg Milchzucker
7,50 mg Talk
0,75 mg Aerosil (R) (chemisch reine Kieselsäure in submikroskopisch feiner Verteilung)
2,25 mg Kartoffelstärke (als 6%iger Kleister).

Beispiel 11

In üblicher Weise werden Dragees folgender Zusammensetzung hergestellt:

10,00 mg 7-Ethyl-7-(3-methoxyphenyl)-4-methyl-hexahydro-1,4-oxazepin-hydrochlorid
50,00 mg Kernmasse
40,00 mg Verzuckerungsmasse.

Die Kernmasse besteht aus 9 Teilen Maisstärke, 3 Teilen Milchzucker und 1 Teil Luviskol(R) VA 64 (Vinylpyrrolidon-Vinylacetat-Milchpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 12

Es werden 5 g 7-Ethyl-7-(3-methoxyphenyl)-4-methyl-hexahydro-1,4-oxazepin-hydrochlorid in 2,0 l Wasser gelöst, mit Kochsalz isotonisch eingestellt und in 2 ml fassende Ampullen steril abgefüllt.

**Patentansprüche für die Vertragsstaaten: BE, CH, DE, FR, GB, IT, LU, NL und SE**

1. Hexahydro-1,4-oxazepine der allgemeinen Formel I,

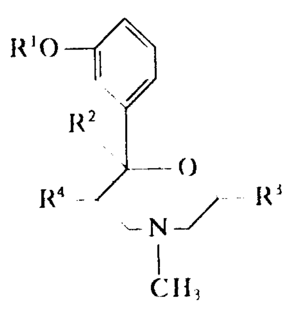

(I)

worin

| | |
|---|---|
| $R^1$ | ein Wasserstoffatom oder eine Alkyl- oder Acylgruppe mit jeweils 1 bis 4 Kohlenstoffatomen, |
| $R^2$ | eine Alkylgruppe mit 1 bis 4 Kohlenstoffatomen und |
| $R^3$ und $R^4$ | gleich oder verschieden sind und Wasserstoffatome oder Methylgruppen bedeuten, sowie deren Salze mit physiologisch verträglichen Säuren. |

2. 7-Ethyl-7-(3-methoxyphenyl)-4-methyl-hexahydro-1,4-oxazepin und dessen Stereoisomere.
3. 2,4-Dimethyl-7-ethyl-7-(3-methoxyphenyl)-hexahydro-1,4-oxazepin.
4. 4,6-Dimethyl-7-ethyl-7-(3-methoxyphenyl)-hexahydro-1,4-oxazepin.
5. 7-Ethyl-7-(3-hydroxyphenyl)-4-methyl-hexahydro-1,4-oxazepin.
6. 7-(3-Acetoxyphenyl)-7-ethyl-4-methyl-hexahydro-1,4-oxazepin.
7. Verfahren zur Herstellung von Hexahydro-1,4-oxazepinen der allgemeinen Formel I gemäß Anspruch 1, dadurch gekennzeichnet, daß man eine Verbindung der allgemeinen Formel II,

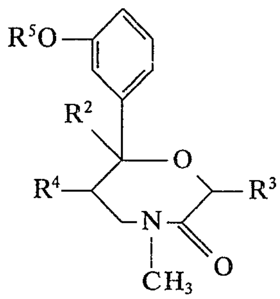

(II)

worin

$R^5$ eine Alkylgruppe mit 1 bis 4 Kohlenstoffatomen bedeutet und
$R^2$, $R^3$ und $R^4$ die in Anspruch 1 genannte Bedeutung haben,

reduziert und anschließend gegebenenfalls die Alkylgruppe $R^5$ gegen ein Wasserstoffatom oder eine Acylgruppe austauscht und die so erhaltenen Verbindungen, falls gewünscht, in ihre Salze mit physiologisch verträglichen Säuren überführt.
8. Arzneimittel enthaltend eine Verbindung gemäß Anspruch 1.
9. Verbindung gemäß Anspruch 1 zur Verwendung bei der Bekämpfung von Schmerzzuständen.

**Patentanspruch für den benannten Vertragsstaat: AT**

Verfahren zur Herstellung von Hexahydro-1,4-oxazepinen der allgemeinen Formel I

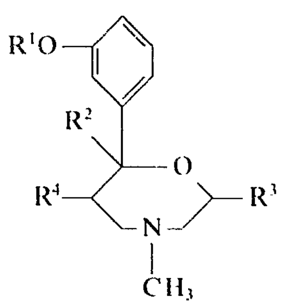

(I)

worin

| | |
|---|---|
| $R^1$ | ein Wasserstoffatom oder eine Alkyl- oder Acylgruppe mit jeweils 1 bis 4 Kohlenstoffatomen, |
| $R^2$ | eine Alkylgruppe mit 1 bis 4 Kohlenstoffatomen und |
| $R^3$ und $R^4$ | gleich oder verschieden sind und Wasserstoffatome oder Methylgruppen bedeuten, sowie deren Salzen mit physiologisch verträglichen Säuren, |

dadurch gekennzeichnet, daß man eine Verbindung der allgemeinen Formel II,

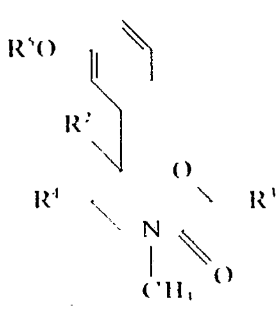

(II)

worin

R⁵ eine Alkylgruppe mit 1 bis 4 Kohlenstoffatomen bedeutet und
$R^2$, $R^3$ und $R^4$ die vorstehende Bedeutung haben,

reduziert und anschließend gegebenenfalls die Alkylgruppe $R^5$ gegen ein Wasserstoffatom oder eine Acylgruppe austauscht und die so erhaltenen Verbindungen, falls gewünscht, in ihre Salze mit physiologisch verträglichen Säuren überführt.

**Claims for the contracting states BE, CH, DE, FR, GB, IT, LU, NL, SE**

1. A hexahydro-1,4-oxazepine of the general formular I

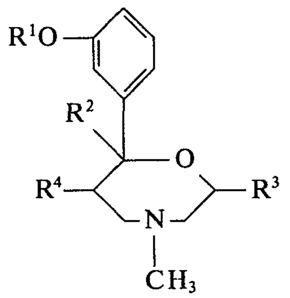

(I)

where

$R^1$ is hydrogen, or alkyl or acyl each of 1 to 4 carbon atoms,
$R^2$ is alkyl of 1 to 4 carbon atoms, and
$R^3$ and $R^4$ are identical or different and each is hydrogen or methyl,

and its salts with physiologically acceptable acids.

2. 7-Ethyl-7-(3-methoxyphenyl)-4-methyl-hexahydro-1,4-oxazepine and its stereoisomers.

3. 2,4-Dimethyl-7-ethyl-7-(3-methoxyphenyl)-hexahydro-1,4-oxazepine.

4. 4,6-Dimethyl-7-ethyl-7-(3-methoxyphenyl)-hexahydro-1,4-oxazepine.

5. 7-Ethyl-7-(3-hydroxyphenyl)-4-methyl-hexahydro-1,4-oxazepine.

6. 7-(3-Acetoxyphenyl)-7-ethyl-4-methyl-hexahydro-1,4-oxazepine.

7. A process for the preparation of a hexahydro-1,4-oxazepine of the general formula I as claimed in claim 1, characterized in that a compound of the general formula II

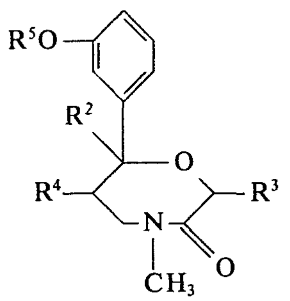

(II)

where

$R^5$ is alkyl of 1 to 4 carbon atoms, and
$R^2$, $R^3$ and $R^4$ have the above meanings,

is reduced and thereafter, if desired, the alkyl group $R^5$ is replaced by hydrogen or acyl, and the compound thus obtained is converted, if desired, to a salt with a physiologically acceptable acid.

8. Drugs containing a compound as claimed in claim 1.

9. A compound as claimed in claim 1 for use in relieving pain.

**Claim for the contracting state AT**

A process for the preparation of a hexahydro-1,4-oxazepine of the general formula I

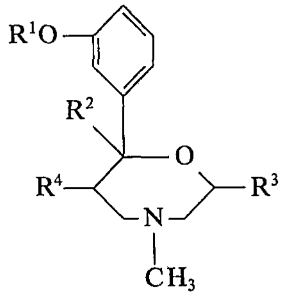

(I)

where

| | |
|---|---|
| $R^1$ | is hydrogen, or alkyl acyl each of 1 to 4 carbon atoms, |
| $R^2$ | is alkyl of the 1 to 4 carbon atoms, and |
| $R^3$ and $R^4$ | are identical or different and each is hydrogen or methyl, |

and its salts with physiologically acceptable acids, characterized in that a compound of the general formula II

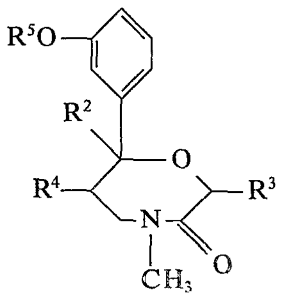

(II)

where

$R^5$ is alkyl of 1 to 4 carbon atoms, and
$R^2$, $R^3$ and $R^4$ have the above meanings,

is reduced and thereafter, if desired, the alkyl group $R^5$ is replaced by hydrogen or acyl, and the compound thus obtained is converted, if desired, to a salt with a physiologically acceptable acid.

**Revendications pour les Etats contractant BE, CH, DE, FR, GB, IT, LU, NL, SE**

1. Hexahydro-1,4-oxazépines de formule générale I

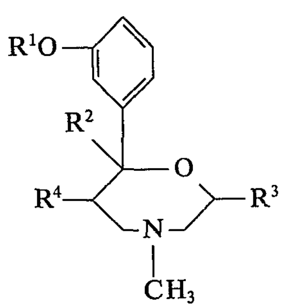

(I)

dans laquelle

| | |
|---|---|
| $R^1$ | est un atome d'hydrogène ou un groupe alkyle ou acyle ayant 1 à 4 atomes de carbone, |
| $R^2$ | un groupe alkyle ayant 1 à 4 atomes de carbone et |
| $R^3$ et $R^4$ | sont identiques ou différents et représentent des atomes d'hydrogène ou des groupes méthyle, |

ou leurs sels avec des acides compatibles physiologiquement.

2. 7-éthyl-7-(3-méthoxyphényl)-4-méthyl-hexahydro-1,4-oxazépine et ses stéréoisomères.
3. 2,4-diméthyl-7-éthyl-7-(3-méthoxyphényl)-hexahydro-1,4-oxazépine,
4. 4,6-diméthyl-7-éthyl-7-(3-méthoxyphényl)-hexahydro-1,4-oxazépine,
5. 7-éthyl-7-(3-hydroxyphényl)-4-méthyl-hexahydro-1,4-oxazépine,
6. 7-(3-acétoxyphényl)-7-éthyl-4-méthyl-hexahydro-1,4-oxazépine,
7. Procédé de préparation d'hexahydro-1,4-oxazépines selon la revendication 1, caractérisé par le fait que l'on réduit un composé de formule générale II

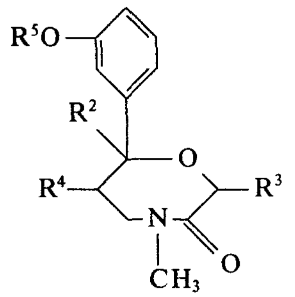

(II)

dans laquelle

$R^5$ présente un groupe alkyle ayant 1 à 4 atomes de carbone et
$R^2$, $R^3$ et $R^4$ ont la signification précédente,

et ensuite on échange éventuellement le groupe alkyle $R^5$ pour un atome d'hydrogène ou un groupe acyle et l'on transforme les composés ainsi obtenus, si on le souhaite, en leurs sels avec des acides compatibles physiologiquement.

8. Médicament contenant un composé selon la revendication 1.
9. Composés selon la revendication 1 pour l'utilisation pour la lutte contre les douleurs.

**Revendication pour l'Etat contractant AT**

Procédé de préparation d'hexahydro-1,4-oxazépines de formule générale I

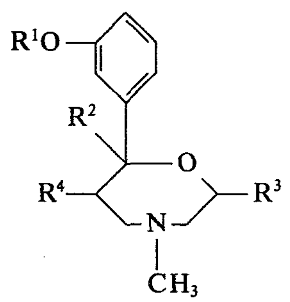

(I)

dans laquelle

$R^1$ est un atome d'hydrogène ou un groupe alkyle ou acyle ayant 1 à 4 atomes de carbone,
$R^2$ un groupe alkyle ayant 1 à 4 atomes de carbone et
$R^3$ et $R^4$ sont identiques ou différents et représentent des atomes d'hydrogène ou des groupes méthyle,

ou leurs sels avec des acides compatibles physiologiquement, caractérisé par le fait que l'on réduit un composé de formule générale II

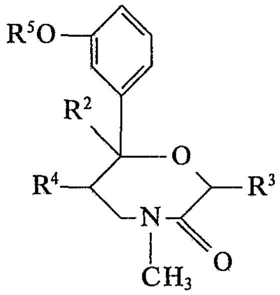

(II)

dans laquelle

$R^5$ représente un groupe alkyle ayant 1 à 4 atomes de carbone et
$R^2$, $R^3$ et $R^4$ ont la signification précédente,

et ensuite on échange éventuellement le groupe alkyle $R^5$ pour un atome d'hydrogène ou un groupe acyle et l'on transforme les composés ainsi obtenus, si on le souhaite, en leurs sels avec des acides compatibles physiologiquement.